# EUROPEAN PATENT APPLICATION

(11) **EP 2 327 776 A1**
(43) Date of publication of application: **01.06.2011**
(21) Application number: 09306158.8
(22) Date of filing: 30.11.2009
(51) Int. Cl.: C12N 9/88, C12P 7/64, C12R 1/645

(54) **Method for the production of Very Long Chain Fatty Acids (VLCFA) by fermentation with a recombinant Yarrowia sp**

(71) Applicant: INSTITUT NATIONAL DE LA RECHERCHE AGRONOMIQUE, 75007 Paris (FR)
(72) Inventor: The designation of the inventor has not yet been filed
(74) Representative: Tetaz, Franck Claude Edouard

(57) **Abstract**

The present invention concerns a method for the production of Very Long Chain Fatty Acids (VLCFA) by fermentation, comprising culturing a recombinant strain of a *Yarrowia* sp. comprising a heterologous gene coding for a hydroxyacyl-CoA dehydratase, under control of regulatory elements allowing expression of the said heterologous gene in the said *Yarrowia* sp.

The invention also concerns the recombinant *Yarrowia* sp.

## Description

### INTRODUCTION

The present invention concerns a method for the production of Very Long Chain Fatty Acids (VLCFA) by fermentation, comprising culturing a recombinant strain of a *Yarrowia* sp. comprising a heterologous gene coding for a hydroxyacyl-CoA dehydratase, under control of regulatory elements allowing expression of the said heterologous gene in the said *Yarrowia* sp.

The invention also concerns the recombinant *Yarrowia* sp.

### BACKGROUND OF THE INVENTION

Living organisms synthesize a vast array of different fatty acids which are incorporated into complex lipids. These complex lipids represent both major structural component membranes, and are a major storage product in both plants and animals.

Very-long-chain fatty acids (VLCFAs) are components of eukaryotic cells and are composed of 20 or more carbons in length (i.e. C18). VLCFAs are involved in many different physiological functions in different organisms. They are abundant constituents of some tissues like the brain (myelin) or plant seed (storage triacylglycerols, TAGs). VLCFAs are components of the lipid barrier of the skin and the plant cuticular waxes. The long acyl chain of certain VLCFAs is necessary for the high membrane curvature, found for instance in the nuclear pore. VLCFAs are also involved in the secretory pathway for protein trafficking and for the synthesis of GPI lipid anchor. Finally, VLCFAs are components of sphingolipids that are both membrane constituents and signalling molecules.

VLCFA are fatty acids with an acyl chain longer than C18. Polyinsaturated, they are considered as important nutritional components of the human diet mainly as Eicosapentaenoic acid (EPA) or Docosahexaenoíc acid (DHA). Unsaturated, VLCFA are also of industrial interest since they act as detergent or lubricants. VLCFA are synthesized by the sequential addition of two carbons through four successive enzymatic reactions gathered in the endoplasmic reticulum within a protein complex named elongase complex. The first step of fatty elongation is the condensation of a long chain acyl-CoA with a malonyl-CoA by the 3-keto-acyl-CoA synthase (KCS or condensing enzymes). The resulting 3-keto-acyl-CoA is then reduced by a 3-keto-acyl-CoA reductase (KCR) generating a 3-hydroxy-acyl-CoA. The third step is the dehydration of the 3-hydroxy-acyl-CoA by a 3-hydoxy-acyl-CoA dehydratase (HCD) to an trans-2,3-enoyl-CoA which is finally reduced by the trans 2,3-enoyl-CoA reductase (ECR) to yield a two carbon elongated acyl-CoA. The last three enzymes are referred as core enzymes since they are not involved in acyl-CoA specificity. Once acyl-CoA have been elongated from the elongase complex, they can be incorporated into different lipid classes, like phospholipids, triacylglycerols, sphingolipids and specific lipids like plant epicuticular waxes.

*Yarrowia lipolytica* is considered as an oleagenous yeast because this yeast can accumulates more than 50% of its dry weight as lipids but also is able to use efficiently lipids as carbon source (Beopoulos & al. 2009). The complete sequencing of its genome as well as the development of molecular genetic tools for this yeast has made this organism not only a model for studying the mechanism of lipid accumulation but also a cell factory for oleochemical biotechnology. Recently, it was shown that the combined deletions of the glucose 3-phosphate dehydrogenase *GUT2* and the *POX1-6* genes involved in the β-oxidation led to very high accumulation of lipids, mainly free fatty acids (Beopoulos & al. 2008, US Patent Application N° 08/54786; 11 July 2008). This obese strain accumulated twice and three times more fatty acids than wild type when grown respectively on glucose or oleic acid. Interestingly, these lipids were accumulated in a single large lipid body. *Yarrowia lipolytica* accumulates mainly the long chain fatty acids c18:2, c18: 1 (n-9), c16:1 (n-7) and c16:0. However little information is available on very long chain fatty acids (VLCFA) in *Y. lipolytica.*

It was now found that expressing a heterologous gene coding for a hydroxyacyl-CoA dehydratase in a *Yarrowia sp.* and particularly *Yarrowia lipolytica* had a direct impact on the strain's production of fatty acids and VLCFA, in terms of quality and/or quantity.

### BRIEF DISCLOSURE OF THE INVENTION

The present invention concerns a recombinant strain of a *Yarrowia* sp., comprising a heterologous gene coding for a hydroxyacyl-CoA dehydratase, under control of regulatory elements allowing expression of the said heterologous gene in the *Yarrowia* sp.

The gene coding for the hydroxyacyl-CoA dehydratase is particularly selected among the group consisting of genes of plant sp. coding for a hydroxyacyl-CoA dehydratase, functional homologues and fragments thereof. The gene of plant sp. is advantageously selected among the genes coding for an hydroxyacyl-CoA dehydratase from *Arabidopsis thaliana, Vitis vinifera, Oryza sativa, Brassica rapa, Hyacinthus orientalis, Ostreacoccus lucimarinus, Chlamydomonas reinhardtii, Brassica napus, Raphanus sativus,* and *Brassica oleracea* and more particularly the gene PAS2 from *Arabidopsis thaliana.*

The invention also concerns a method for the production of Very Long Chain Fatty Acids (VLCFA) by fermentation, comprising culturing a recombinant strain of the invention in an appropriate culture medium and recovering the VCLFA from the strains and/or the medium.

The fatty acids produced by the said method are also parts of the invention.

### DETAILLED DISCLOSURE OF THE INVENTION

The present invention concerns a recombinant strain of a *Yarrowia* sp., comprising a heterologous gene coding for a hydroxyacyl-CoA dehydratase, under control of regulatory elements allowing expression of the said heterologous gene in the *Yarrowia* sp.

### recombinant strain

According to the invention, the strain is recombinant when it has been genetically modified by means of cellular biology such as gene replacement or plasmid introduction. It may be obtained by directed mutagenesis to introduce a new gene or mutations or new regulatory elements in a gene or to delete an endogenous gene. A recombinant microorganism is not the sole result of random mutagenesis.

When the new gene is introduced in the strain, it may be introduced with an expression plasmid, or integrated in the genome of the strain.

When integrated in the genome of the strain, the gene may be integrated randomly or on a specific site by known methods of gene replacement, like homologous recombination techniques.

The heterologous gene when introduced can comprise the coding sequence under control of the regulatory elements allowing expression of the said heterologous gene in the *Yarrowia* sp. Alternatively, it can comprise the coding sequence which is introduced in the genome of the microorganism under control of existing endogenous regulatory elements, replacing the corresponding endogenous coding sequence which is deleted.

Methods for the modification of a *Yarrowia sp.* particularly to introduce new genes or delete genes are known in the art, including Barth and Gaillardin (1996) and Fickers et al. (2003).

### heterologous gene coding for a hydroxyacyl-CoA dehydratase

The gene coding for the hydroxyacyl-CoA dehydratase is heterologous. According to the invention, a gene is heterologous when it is not found as such in the native strain. It can be a native coding sequence under control of heterologous regulatory elements or a heterologous coding sequence under control of native regulatory elements. It can also be a gene with native components, found in the strain to be modified, but on a plasmid or in a locus in the genome where the same gene is not found in the unmodified strain.

The heterologous gene coding for a hydroxyacyl-CoA dehydratase is particularly selected among the group consisting of genes comprising a coding sequence from a gene of plant sp. coding for a hydroxyacyl-CoA dehydratase, functional homologues and fragments thereof.

Genes of plant sp. coding for a hydroxyacyl-CoA dehydratase are known in the art and includes particularly selected among genes from *Vitis vinifera* (encoding CAN64341.1 hypothetical protein), *Oryza sativa* (CAD39891.2, EAY72548.1 hypothetical protein OsI_000395, EAZ30025.1 hypothetical protein OsJ_013508 and BAD61107.1 tyrosine phosphatase-like), *Brassica rapa* (AAZ66946.1), *Hyacinthus orientalis* (AAT08740.1 protein tyrosine phosphatase), *Ostreacoccus lucimarinus* (XP_001420997.1 predicted protein and XP_001422898.1 predicted protein), *Chlamydomonas reinhardtii* (EDP01055.1 predicted protein), and also from *Brassica napus, Raphanus sativus, Brassica oleracea.*

In a preferred embodiment of the invention, the heterologous gene is the gene PAS2 from *Arabidopsis thaliana,* registered in UniGene databank under number NP_196610.2, also known as F12B17.170; F12B17_170; PASTICCINO 2; PEP; and PEPINO.

In another specific embodiment of the invention, the heterologous gene is the *PHS1* gene from *Saccharomyces cerevisiae,* registered in gene databanks under number NP_012438.1, functional homologues and fragments thereof.

When the coding sequence of the heterologous gene is from another origin, it can be indeed recoded with preferred codon usages known for *Yarrowia* sp. The skilled person knows the preferred codon used in *Yarrowia* sp and how to prepare such a recoded coding sequence.

According to the invention, "functional homologues" are genes sharing homology with the heterologous gene coding for the hydroxyacyl-CoA dehydratase, or a gene encoding for a protein sharing homology with the protein encoded by the heterologous gene coding for a hydroxyacyl-CoA dehydratase.

A protein sharing homology with the protein encoded by the gene coding for a hydroxyacyl-CoA dehydratase may be obtained from plants or may be a variant or a functional fragment of a natural protein originated from plants.

The term "variant or functional fragment of a natural protein" means that the amino-acid sequence of the polypeptide may not be strictly limited to the sequence observed in nature, but may contain additional amino-acids. The term "a fragment" means that the sequence of the polypeptide may include less amino-acid than the original sequence but still enough amino-acids to confer hydroxyacyl CoA dehydratase activity. It is well known in the art that a polypeptide can be modified by substitution, insertion, deletion and/or addition of one or more amino-acids while retaining its enzymatic activity. For example, substitution of one amino-acid at a given position by a chemically equivalent amino-acid that does not affect the functional properties of a protein are common. For the purpose of the present invention, substitutions are defined as exchanges within one of the following groups :
■Small aliphatic, non-polar or slightly polar residues : Ala, Ser, Thr, Pro, Gly
■Polar, negatively charged residues and their amides : Asp, Asn, Glu, Gln
■ Polar, positively charged residues : His, Arg, Lys
■ Large aliphatic, non-polar residues : Met, Leu, Ile, Val, Cys
■ Large aromatic residues : Phe, Tyr, Trp.

Thus, changes that result in the substitution of one negatively charged residue for another (such as glutamic acid for aspartic acid) or one positively charged residue for another (such as lysine for arginine) can be expected to produce a functionally equivalent product.

The positions where the amino-acids are modified and the number of amino-acids subject to modification in the amino-acid sequence are not particularly limited. The man skilled in the art is able to recognize the modifications that can be introduced without affecting the activity of the protein. For example, modifications in the N- or C-terminal portion of a protein may be expected not to alter the activity of a protein under certain circumstances.

The term "variant" refers to polypeptides submitted to modifications such as defined above while still retaining the original enzymatic activity.

According to the invention, the polypeptide having an hydroacyl-CoA dehydratase enzymatic activity may comprise a sequence having at least 30 % of homology with the sequence of PAS2, preferentially at least 50% of homology, and more preferentially at least 70% of homology.

Methods for the determination of the percentage of homology between two protein sequences are known from the man skilled in the art. For example, it can be made after alignment of the sequences by using the software CLUSTALW available on the website http://www.ebi.ac.uk/clustalw/ with the default parameters indicated on the website. From the alignment, calculation of the percentage of identity can be made easily by recording the number of identical residues at the same position compared to the total number of residues. Alternatively, automatic calculation can be made by using for example the BLAST programs available on the website http://www.ncbi.nlm.nih.gov/BLAST/ with the default parameters indicated on the website.

### regulatory elements allowing expression of the heterologous gene in the Yarrowia sp.

Such regulatory elements are well known in the art and include the POX2 promoter from acyl-CoA oxidase 2, the ICL promoter from Isocitrate dehydrogenase, the Promoter Hp4d, the Promoter GPD and GPM, the Promoter FBP and the Promoter XPR2. Said promoters are known in the art and disclosed, inter alia in Juretzek & al. (2000), Madzak & al. (2004), Madzak & al. (2000), US 7 259 255, US 7 202 356 and Blanchin-Roland et al (1994).

### Yarrowia sp.

According to the invention, any strain of a *Yarrowia* sp. may be transformed and used in the method of the invention. Preferably, the strain of *Yarrowia* sp. belongs to the genus *Yarrowia lipolytica.*

Strains of the genus *Yarrowia lipolytica* are well known in the art, as well as method for transforming such strains. Constructs comprising a coding region of interest may be introduced into a host cell by any standard technique. These techniques include transformation (e.g., lithium acetate transformation [ Methods in Enzymology, 194:186-187 (1991)]), protoplast fusion, biolistic impact, electroporation, microinjection, or any other method that introduces the gene of interest into the host cell. More specific teachings applicable for oleaginous yeast (i.e., *Yarrowia lipolytica )* include U.S. Pat. Nos. 4,880,741 and 5,071,764.

Strains modified for an improved production of fatty acids have also been disclosed, like strains with very high accumulation of lipids, mainly free fatty acids (US Patent Application N° 08/54786; 11 July 2008), incorporated herein by reference. Such strains may be further modified according to the invention with a heterologous gene coding for a hydroxyacyl-CoA dehydratase.

The recombinant strain of the invention can also comprise deletion of at least one gene involved in the β-oxidation of fatty acids, particularly the deletion of one of the gene POX1 to POX6 coding for an acyl CoA oxidase, particularly the deletion of the six genes POX1-6 coding for the six acyl CoA oxidases And/or one gene involved in the patway of fatty acid and TAG synthesis, particularly the deletion of the gene coding for a glycerol 3-phosphate dehydrogenase.

### Culture of the recombinant strain

The fatty acids and particularly the VLCFA are produced when culturing the recombinant strain by fermentation in an appropriate culture medium.

Culture by fermentation means that the microorganism are developed on a culture medium and produce the VLCFA during this culture step, by transforming the source of carbon of the culture medium. The VLCFA is accumulated with the biomass, in the cells and/or in the medium.

Fermentation is distinct from bioconversion where the culture is used to produce enzymes, further used in a enzymatic conversion process.

### Appropriate culture medium

Culture mediums for *Yarrowia* sp. are well known in the art, including Barth and Gaillardin (1996), Nicaud et al. (2002) and Mauersberger and Nicaud (2002).

Define media for fermentation are particularly disclosed in Leblond & al. (2009) and KR 2009 0029808.

Sucrose media are particularly disclosed in Nicaud & al. (1989).

Appropriate culture mediums are those mediums where the *Yarrowia* sp. can grow and contains all the nutrients allowing growth of the strain and production of VLCFA, particularly a source of carbon.

The source of carbon may be any source of carbon, such as sucrose or other carbohydrates.

### Recovering the VCLFA from the strains and/or the medium

The VCLFA are accumulated with the biomass, in the strains and/or in the culture medium. Recovery of the VCFLA comprises generally steps of cells lysis, filtration and recovery from the medium. The person skilled in the art of fatty acids bioproduction knows how to adapt the usual methods for recovering a fatty acid from the biomass to the method of the invention.

The VCFLA produced with the method of the invention may be used as such, in mixtures of fatty acids produced by the strain of the invention. They can also be further purified and isolated.

### FIGURES

Figure 1 represents the synthetic PAS2 optimized for Yarrowialopolytica expression. (A) Sequence of PAS2Y1 gene and protein. (B) Alignment of Arabidopsis PAS2At with PAS2Y1.
Figure 2 represents PAS2 expression in Yarrowia with a schematic view of the different strains used or created.
Figure 3 represents Effect of PAS2 expression in Yarrowia. (A) Effect of PAS2 expression on Pold growth. (B) Effect of PAS2 expression on JM1367 growth.
Figure 4 shows ratio LCFA/VLCFA with PAS2 expression in Yarrowia.
Figure 5 shows modified LCFA and VLCFA contents with PAS2 expression in Yarrowia. (A) LCFA content. (B) VLCFA content.
Figure 6 shows modified the VLCFA profile with PAS2 expression in Yarrowia.
Figure 7 shows accumulation of new lipids with PAS2 expression in Yarrowia.

### EXAMPLES

### Material and Methods.

The synthetic PAS2 gene (*PAS2^{Yl}*) was synthesised according to *Yarrowia lipolytica* codon usage giving rise to plasmid JME1107. *PAS2^{Yl}* was cloned into plasmid JMP62-POX2-URA3ex (JME803) and into JMP62-TEF-URA3ex (JME1012) as follow: Plasmid JME1107 was digested by BamHI-AvrII and the corresponding fragment carrying PAS2 gene was cloned at the corresponding site of plasmid JME1012 and JME1107, giving rise to plasmid JME1108 (POX2-PAS2) and JME1110 (TEF-PAS2) respectively. Plasmids were digested by NotI and the fragment carrying the expression cassette were used for transformation of *Yarrowia lipolytica* by the lithium acetate method (described in the revue of G. Barth and Gaillardin : (Yarrowia lipolytica, in: Nonconventional Yeasts in Biotechnology A Handbook (Wolf, K., Ed.), Vol. 1, 1996, pp. 313-388. Springer-Verlag). Transformants were selected onto YNBcasa. Typically, about 5 × 10³ transformants were obtained per µg of fragments. Four to height transformants were analysed by PCR with primer pairs 61start/61stop and TEFstart/61stop for clones containing the POX2-PAS2 and TEF-PAS2, respectively. The PCR products were further digested by AvaI unique restriction site in the PAS2 gene.

### Stable expression of PAS2 in Yarrowia lipolytica

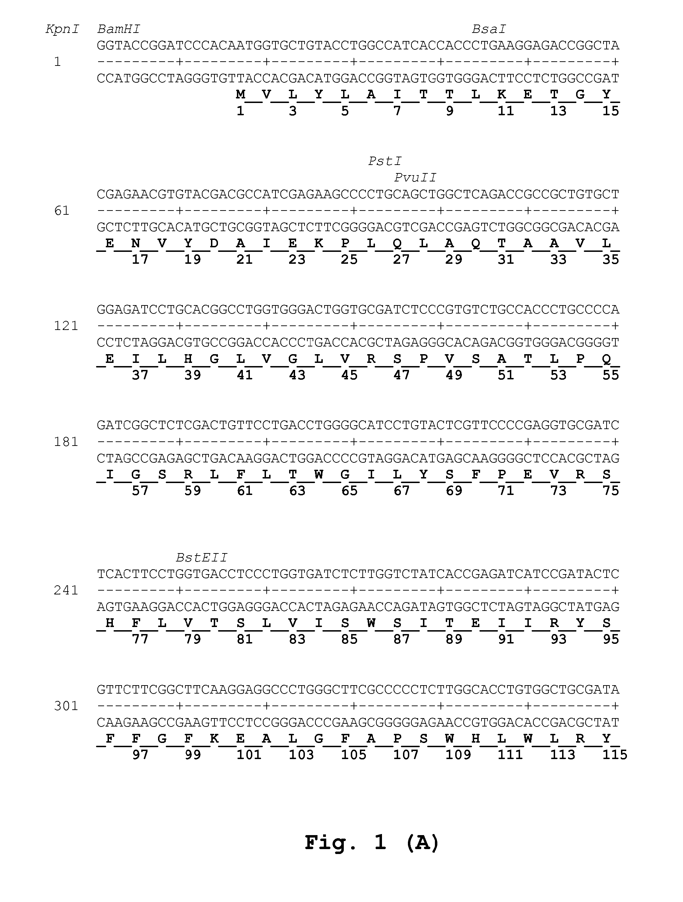

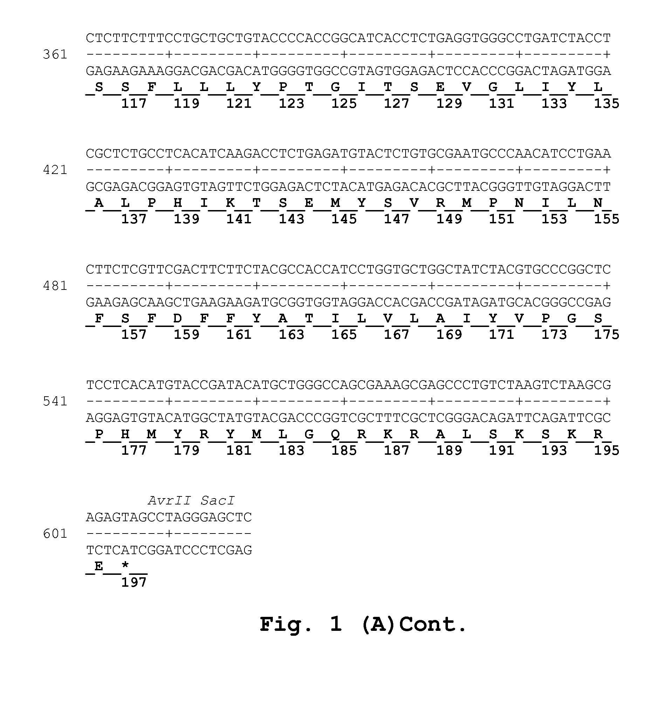

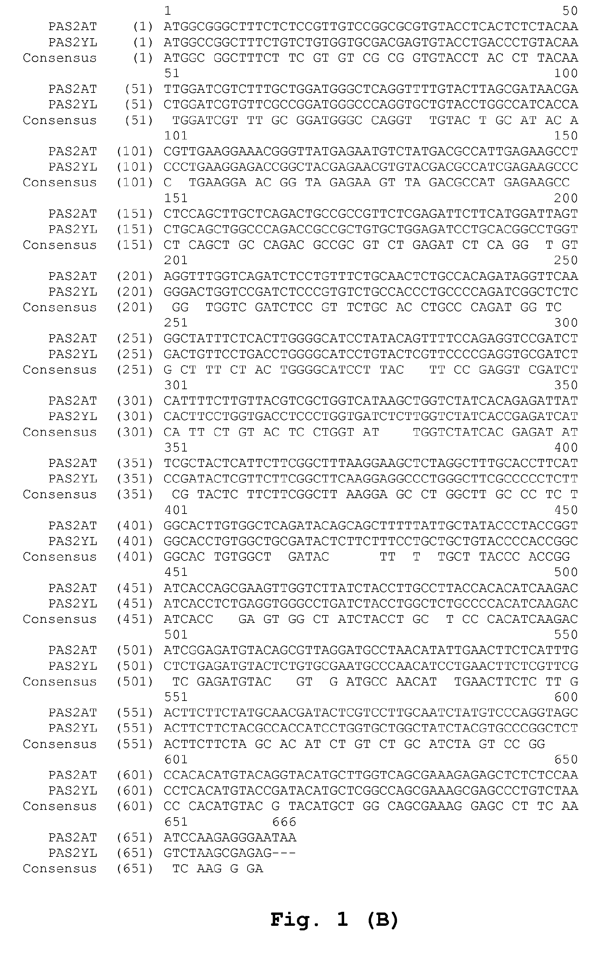

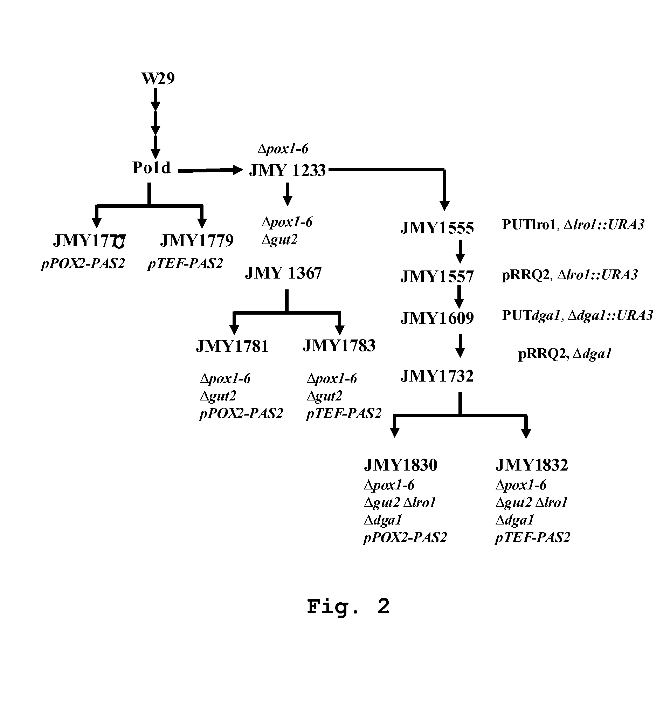

The open reading frame of Arabidopsis PAS2 gene was recoded to improve its expression with Yarrowia codon usage (Fig.1A-B). Two restriction sites was added to facilitate cloning, BamHI and AvrII respectively at the 5' and the 3' end of PAS2 ORF. The new sequence, renamed PAS2^{Yl} was chemically synthetized (GeneArt inc.) and cloned into the two expression vectors JME1110 and JME1108 (Fig.2A-B). The two vectors allow the expression of PAS2 under a constitutive promoter (pTEF, JME1110) or oleic acid inducible promoter (pPOX2, JME1108).

Both constructs were used to transform the wild type strain Pold (JMY195) and the Δg*ut2* Δ*pox1-6* obese strain (JMY1367) (Fig.2C). Transformants were selected on uracil and integration of the expression casette were verified by PCR. Several clones were selected and used for further analysis. However, since POX2 promoter allow strong expression even in absence of inducer, we mainly characterized transformants with JME1108 construct. The strains JMY1777 and JMY1778 are two independent clones of Pold transformed with pPOX2-PAS2. Similarly, JMY1781 and JMY1782 are two independent clones of JMY1367 *(*Δ*gut* Δ*pox1-6)* transformed with pPOX2-PAS2.

### PAS2 expression improves cell growth and leads to lipid body fragmentation

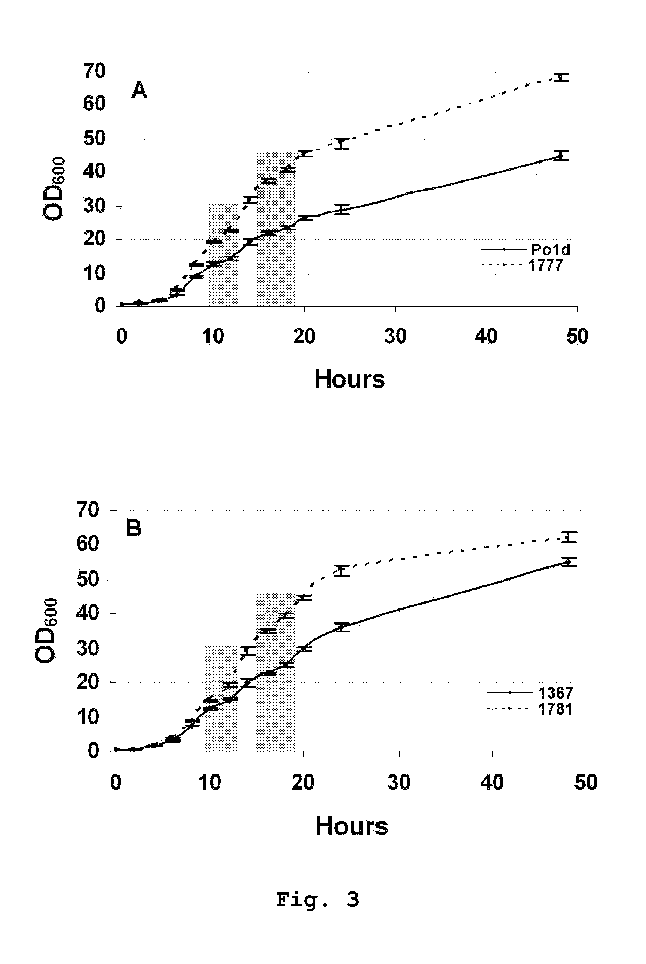

The growth of different PAS2 expressing strains were compared with their untransformed relatives on glucose supplemented media. All the strains were inoculated at OD600 = 0.6 in 30 ml of YPD medium. All the strains showed a lag phase of about 4 to 5 hours. Growth of yeasts follows a bimodal curve with two plateaus at 9 to 12 hours and at 15 to 19 hours after inoculation before to reach the beginning of the stationary phase after 40 hours of culture (Fig.3A-B). Expression of PAS2 in the wild type Pold strain led to 55% biomass increase at the first plateau and even 73% at the second plateau (Fig.2A). A similar effect could be noted in JMY1367 obese strain with 24% at the first plateau and even 55% at the second plateau (Fig.2B). The growth increase was not associated with obvious differences in morphology or cell sizes (Fig.2C).

*Yarrowia lipolytica* is known to accumulate lipids in lipid bodies. It was reported that the obese strain JMY1367 was characterized by fusion of the lipid bodies in a single large structure lipid vesicule. The effect of PAS2 expression on the structure of the lipid bodies was thereby checked by staining the different strains with Nile red. Cells were collected at 48h since stationary stage is characterized with high accumulation of lipids. Pold is characterized by the accumulation of several small lipid bodies and the expression of PAS2 did not modify significantly the size and numbers of the lipid bodies (numbers, not yet defined). However, JMY1367 which is normally characterized by a large and single lipid body showed clear fragmentation of its lipid body into multiple lipid vesicules when expressing PAS2 (Fig.3C).

### PAS2 expression enhances VLCFA levels

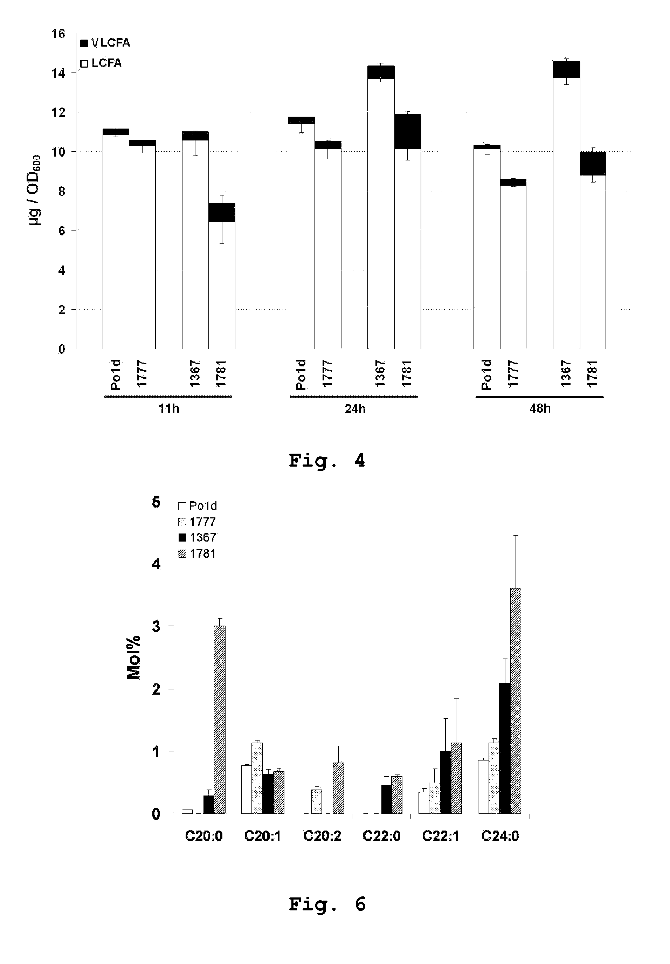

Total lipid content was analysed by gas chromatography of fatty acyl methyl esters (FAMES) in the four strains at 3 different time point of growth curve, at the end of the first growth phase (11h ), at the end of second growth phase (24h) and during stationary phase (48h). As expected the obese strain JMY1367 has a higher fatty acid content compared to wild type Pold with 22% and 40% at 24h and 48h respectively (Fig.4). The expression of PAS2 reduced total fatty acid content at every time point. The strongest reduction was observed at 48h with 20% in Pold background and 46% in JMY1367 background.

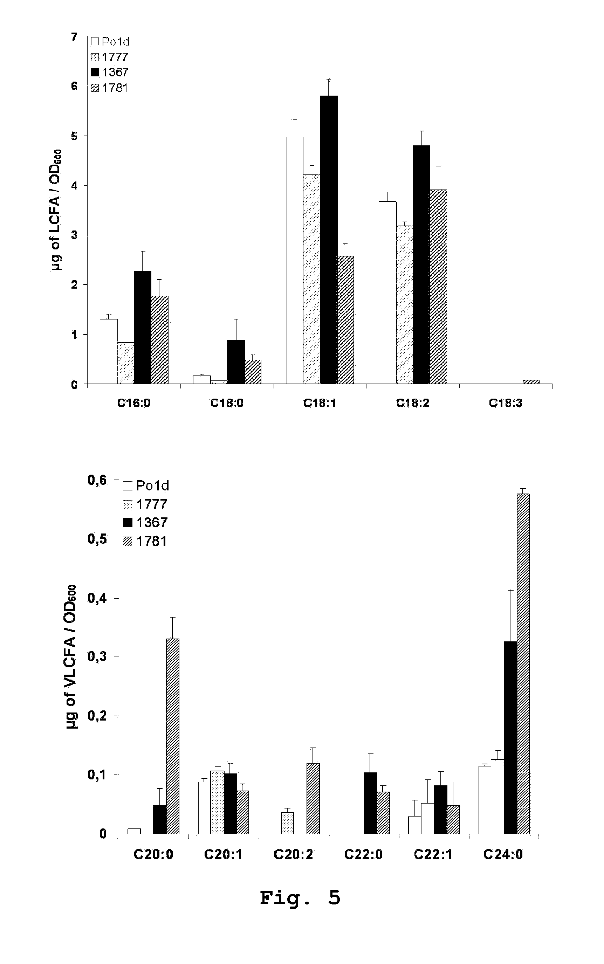

The amount of total long chain fatty acids (LCFA) which represent the most abundant fatty acids of *Yarrowia lipolytica,* was reduced by 18 and 36% in PAS2 expressing strains. Analysis of LCFA showed that all the different classes showed reduced levels upon PAS2 expression except that c18:1, which is one of the most abundant LCFA, was the most affected with for instance 126% reduction at 48h in the obese JMY1367 background (Fig.5). The reduction in total LCFA was effective even at the beginning of the growth curve (11h).

VLCFA represent only minor lipid species in *Yarrowia lipolytica* (2.2-3.2% total fatty acids) (Fig.4). Three major species were significantly accumulated: 24:0, 20:1 and 22:1 representing respectively 0.86, 0.77 and 0.34 % of total fatty acids (Mol%) at 48h of culture (Fig.6). The Δ*gut* Δ*pox1-6* had a clear effect on VLCFA levels since it doubled in 24h of culture (6.53µg/10OD compared to 3.23 in Pold). The main VLCFA involved were c24:0 and c22:1 content that reached respectively 2.09 and 0.99% (Mol%) of total fatty acids. A new VLCFA could be detected as c22:0 reaching 0.45%. The expression of PAS2 in Pold background did not change much the quantity or the nature of VLCFA accumulated. However, the expression of PAS2 in the obese JMY1367 background, increased very significantly VLCFA content. After 24h of culture, JMY1781 accumulated 17.35µg/10OD which was 2.65 and 5.3 fold more than the obese and wild type Pold strains, respectively. The main VLCFA accumulated were c20:0 and c24:0 representing more than half of total VLCFA. Erucic acid c22:1, c22:0, c20:2 were also significantly accumulated in JMY1781.

### PAS2 expression induce the accumulation of new monomethyl branched fatty acids

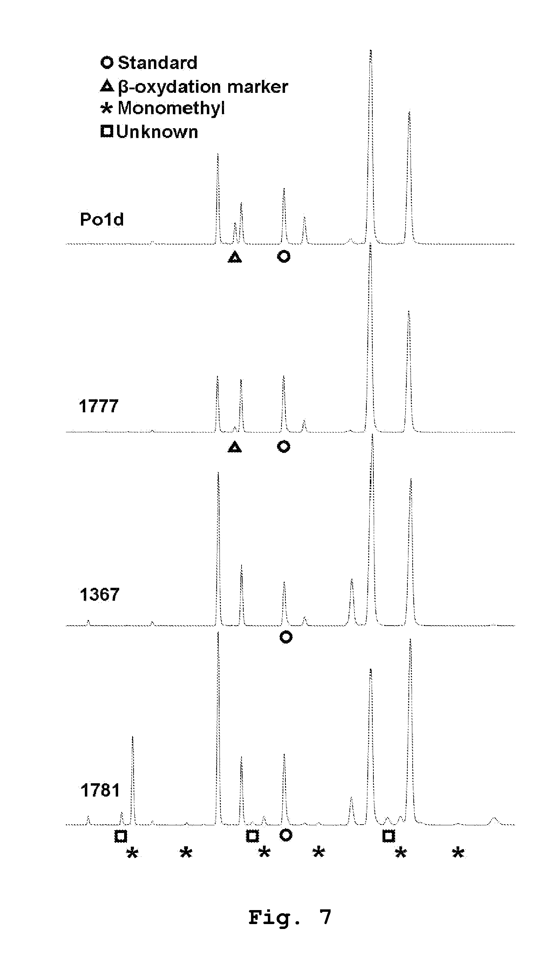

Detail analysis of FAMES revealed that PAS2 expressing strain JMY1781 was accumulating new fatty acids (Fig.7). Mass spectrometry determined that lipids were monomethyl branched fatty acids with even or odd acyl chains (Table III). The JMY1781 showed in particular the presence of c14:0(Me), c15:0(Me), c16:0(Me), c17:0(Me), c18:0(Me) and c19:0(Me) (Fig.7). The compounds were almost undectable in the wild type Pold but also in the obese JMY1367 strains. The tetradecanoic acid, 12 methyl, methyl ester, 14:0(Me), appeared to be highly accumulated (at least to the level of Octadecanoic acid, methyl ester, c18:0). Several other products were accumulated in JMY1781 strain like the peaks at 10.7min, 14.4min, 18.4min and 23min.

### Discussion

The expression of the 3hydroxyacylCoA dehydratase PASTICCINO from Arabidopsis in *Yarrowia lipolytica* led to several innovative traits concerning the use of this yeast as a cell factory for oleochemichal biotechnology.
1- The expression of PAS2^{Y1} improves the growth of two different Yarrowia strains: a wild type Pold but also the Δ*gut* Δ*pox1-6* characterized by high accumulation of fatty acids inside the cell. Moreover, the expression of PAS2 in the latter strain lead to the fragmentation of the lipid bodies in smaller vesicules. The consequence of this change of lipid body size for lipid extraction needs to be further evaluated. The possibility that PAS2 modifies lipid secretion needs also to be checked.
2- The expression of PAS2^{Y1} causes a very significant increase in VLCFA accumulation. The levels of VLCFA are still far from being used directly for industrial production but it opens new avenues for the genetic engineering of *Y. lipolytica.* The obvious experiments to perfom would be to co-express in Yarrowia the other genes of the elongase complex described in the introduction. Since expression of an Arabidopsis gene has proved to be efficient in changing VLCFA homeostasis, it is proposed to use in the future the other elongase genes from plants.
3- The expression of PAS2^{Y1} is sufficient for the accumulation of new monomethyl branched fatty acids of different chain length (even and odd numbered) ranging from c14 to at least c19. The industrial use of branched fatty acid is limited since they are rare in nature and can be only synthesized chemically. The physical properties of branched-chain fatty acids are very different from straight-chain acids. The introduction of a branch lowers the melting point and surface tension but also improves the stability to oxidation. They are found in many products including cosmetics, lubricants, fabric softener etc. For biofuels, esters of fatty compounds obtained from branched alcohols have improved low-temperature properties compared to those derived from straight-chain alcohols. The present work shows that the branched esters are fully competitive in terms of ignition characteristics to the esters derived from straight-chain alcohols. Therefore, from a technical standpoint, they appear to be preferable to the methyl esters now commonly used as biodiesel. Disadvantages of the branched esters are the higher price of the alcohol and the changes needed in the (trans)esterification process yielding the branched esters. The possibility to produce directly branched fatty acids that can be turned into esters could thus be of interest.

**TABLE 1. Strains and plasmids used in this study**

| Strain (host strain) | Plasmid, genotype | Reference or source |
|---|---|---|
| | *E coli* strains | |
| DH5α | Φ80d*lac*ZΔm15, *rec*A1, *end*A1, *gyr*A96, *thi*-1, *hsd*R17 (rₖ-, mₖ+), *sup*E44, *rel*A1*, deo*R, Δ(*lac*ZYA-*arg*F)U169 | Promega |
| JME461 (DH5α) | pRRQ2(cre ARS68 LEU2 in pBluescript II KS+) | Fickers and al 2003 |
| | | |
| JME803 (DHSα) | JMP62-*URA3ex* , expression vector with the excisable *URA3ex* marker and the POX2 promoter. | Nicaud and al, 2002 |
| JME1012 (DH5α) | JMP62-*URA3ex* , expression vector with the excisable *URA3ex* marker and the TEF promoter. | This work |
| JME1107 (DH5α) | Synthetic *PAS2* gene optimised with the codon usage of *Y. lipolytica.* | Geneart |
| | | |
| JME1108 (DH5α) | *PAS2-URA3,* expression vector with the *URA3ex*marker under the pPOX2 promoter inducible by oleic acid. | This work |
| JME1110 (DH5α) | *PAS2-URA3,* expression vector with the *URA3ex*marker under a constitutive promoter pTEF | This work |

| | *Y. lipolytica* strains | |
|---|---|---|
| JMY399, W29 | *MATA, wild-type* | Barth and Gaillardin, 1996 |
| JMY195, Pold | *MATA ura*3-302 *leu2-270 xpr*2-322 | Barth and Gaillardin, 1996 |
| MTLY95a, JMY1233 | *MATA ura*3-302 *xpr*2-322 *Δleu2 Δpox1-6* | Thevenieau *et al,* 2004 |
| JMY1367 | *MATA ura*3-302 *xpr*2-322, *Δleu2 Δpox1-6 Δgut2* | Beopoulos, 2008 |
| JMY1732 | *MATA ura*3-302 *xpr*2-322 *Δleu2 Δpox1-6 Δlrol Δdgal* | This work |
| JMY 1777 | Pold, JMP62- *URA3ex*-pPOX2-*PAS2* | This work |
| JMY 1779 | Pold, JMP62- *URA3ex*-pTEF-*PAS2* | This work |
| JMY 1781 | JMY1367, JMP62- *URA3ex*-pPOX2-*PAS2* | This work |
| JMY 1783 | JMY1367, JMP62- *URA3ex*-pTEF-*PAS2* | This work |
| JMY 1830 | JMY 1732, JMP62- *URA3ex*-pPOX2-*PAS2* | This work |
| JMY 1832 | JMY 1732, JMP62- *URA3ex*-pTEF-*PAS2* | This work |

**TABLE II. Primers used in this study**

| Primers | Sequence (5'→ 3')^{a} | Restriction site, introduced |
|---|---|---|
| LRO1-ver1 | CCACGGAGACTGGAACAGATGTCGG | |
| LRO1-P1 | GGATCCCAGTGCTCTAGACTGTC | |
| LRO1-P2 | GCTAGGGATAACAGGGTAATGCGCGGTAGCTGAGACATGTCGCGTG | Iscel |
| LRO1-T1 | GCATTACCCTGTTATCCCTAGCGCGTTCGTCCTCTCATGATTCC | Iscel |
| LRO1-T2 | CCAAACATAGTCATTTGCGGATCC | |
| LRO1-ver2 | CCAAGGGACCGTCTGGCCAAAC | |
| DGA1-ver1 | CGGACACCTCTTTTATGCTGCGGGC | |
| DGA1-P1 | GGCGGATCCTGGTGCATTTTTGC | |
| DGA1-T1 | GCTAGGGATAACAGGGTAATGCGCAAACTCATCTGGGGGAGATCC | Iscel |
| DGA1-P2 | GCATTACCCTGTTATCCCTAGCGAGCTTATCAGTCACGGTCCACG | Iscel |
| DGA1-T2 | CCATAGAGGTGTCCCCAAACG | |
| DGA1-ver2 | CCCCCAAGCATACCGACCGTCGC | |
| 61start^{b} | CTTATATACCAAAGGGATGGGTC | |
| 61Stop^{b} | GTAGATAGTTGAGGTAGAAGTTG | |
| TEFstart^{b} | GGGTATAAAAGACCACCGTCC | |

| | | |
|---|---|---|
| ^{a}underlined sequences correspond to introduced restriction sites | | |

### References

● Barth and Gaillardin. : (Yarrowia lipolytica, in: Nonconventional Yeasts in Biotechnology A Handbook (Wolf, K., Ed.), Vol. 1, 1996, pp. 313-388. Springer-Verlag).
● Beopoulos, A., Mrozova, Z., Thevenieau, F., Le Dall, M.T., Hapala, I., Papanikolaou, S., Chardot, T., and Nicaud, J.M. (2008). Control of lipid accumulation in the yeast Yarrowia lipolytica. Appl Environ Microbiol 74, 7779-7789.
● Beopoulos, A., Cescut, J., Haddouche, R., Uribelarrea, J.L., Molina-Jouve, C., and Nicaud, J.M. (2009). Yarrowia lipolytica as a model for bio-oil production. Prog Lipid Res 48, 375-387.
● Blanchin-Roland et al., Two Upstream Activation Sequences Control the Expression of the XPR2 Gene in the Yeast Yarrowia lipolytica, Molecular and Cellular Biology, vol. 14(1):327-338, 1994.
● Fickers P., Le Dall M.T., Gaillardin C. Thonart P. Nicaud J-M. (2003) New disruption cassettes for rapid gene disruption and marker rescue in the yeast Yarrowia lipolytica. J. Microbiol. Methods 55/3:727-737.
● Juretzek T, Wang H., Nicaud, J-M., ,Mauersberger S, Barth G. (2000). Comparison of promoters suitable for regulated overexpression of β-galactosidase in the alkane-utilizing yeast Yarrowia lipolytica. Biotechnol. Bioprocess Eng. 5:320-326.
● Leblond, Y., A. Marty, N. Mouz, and J. L. Uribelarrea. 2009. Method for producing lipase, transformed Yarrowia lipolytica cell capable of producing said lipase and their uses.
● Madzak, C., Gaillardin, C. and Beckerich, J.M. (2004). J. Biotechnol. 109: 63 - 81. (review).
● Madzak, C., Treton, B. and Blanchin-Roland, S. (2000). J. Mol. Microbiol. Biotechnol. 2: 207 - 216.
● Mauersberger, S., Nicaud, J-M (2002) Chapter 56. Tagging of genes by insertional mutagenesis in the yeast Yarrowia lipolytica. In: Laboratory Manual on Nonconventional Yeasts in Genetics, Biochemistry and Biotechnology.
● Nicaud J-M., Fabre E., Gaillardin C. (1989) Expression of invertase activity in Yarrowia lipolytica and its use as a selective marker. Cur. Genet. 16 :253-260.
● Nicaud, J-M, Madzak, C., van den Broek, P., Gysler, C., Duboc, P., Niederberger, P. Gaillardin, C. (2002) Protein expression and secretion in the yeast Yarrowia lipolytica. FEMS Yeast Research 2/3:371-379.
● Wolf K, Breunig K, Barth G (eds), Springer, Berlin Heidelberg-New York, pp 343-356. (Buchbeitrag).

## Claims

1. A recombinant strain of a *Yarrowia* sp., wherein it comprises a heterologous gene coding for a hydroxyacyl-CoA dehydratase, under control of regulatory elements allowing expression of the said heterologous gene in the *Yarrowia* sp.

2. The recombinant strain of claim 1, wherein the gene coding for the hydroxyacyl-CoA dehydratase is selected among the group consisting of genes of plant sp. coding for a hydroxyacyl-CoA dehydratase, functional homologues and fragments thereof.

3. The recombinant strain of claim 2, wherein the gene of plant sp. is selected among the genes coding for a hydroxyacyl-CoA dehydratase from *Arabidopsis thaliana, Vitis vinifera, Oryza sativa, Brassica rapa, Hyacinthus orientalis, Ostreacoccus lucimarinus, Chlamydomonas reinhardtii, Brassica napus, Raphanus sativus,* and *Brassica oleracea.*

4. The recombinant strain of claim 3, wherein the gene of plant sp. is the gene PAS2 from *Arabidopsis thaliana.*

5. The recombinant strain of one of claims 1 to 3, wherein the strain of *Yarrowia* sp. belongs to the genus *Yarrowia lipolytica.*

6. The recombinant strain of one of claims 1 to 5, wherein the strain comprises deletion of at least one gene involved in the β-oxidation of fatty acids.

7. The recombinant strain of claim 6, wherein it comprises the deletion of the gene coding for a glucose 3-phosphate dehydrogenase and/or the gene *POXI-6.*

8. A method for the production of Very Long Chain Fatty Acids (VLCFA) by fermentation, comprising culturing a recombinant strain of one of claims 1 to 7 in an appropriate culture medium and recovering the VCLA from the strains and/or the medium.

9. A VLCFA composition obtained by the method of claim 8.


SEQUENCE LISTING

<110>  Institut National de la Recherche Agronomique (INRA)

<120>  Method for the production of Very Long Chain Fatty Acids (VLCFA)
       by fermentation with a recombinant Yarrowia sp

<130>  D28047

<160>  20

<170>  PatentIn version 3.3

<210>  1
<211>  666
<212>  DNA
<213>  artificial

<220>
<223>  Recoded Arabidopsis PAS2 gene (PAS2AT)

<400>  1
atggcgggct ttctctccgt tgtccggcgc gtgtacctca ctctctacaa ttggatcgtc      60

tttgctggat gggctcaggt tttgtactta gcgataacga cgttgaagga aacgggttat     120

gagaatgtct atgacgccat tgagaagcct ctccagcttg ctcagactgc cgccgttctc     180

gagattcttc atggattagt aggtttggtc agatctcctg tttctgcaac tctgccacag     240

ataggttcaa ggctatttct cacttggggc atcctataca gttttccaga ggtccgatct     300

cattttcttg ttacgtcgct ggtcataagc tggtctatca cagagattat tcgctactca     360

ttcttcggct ttaaggaagc tctaggcttt gcaccttcat ggcacttgtg gctcagatac     420

agcagctttt tattgctata ccctaccggt atcaccagcg aagttggtct tatctacctt     480

gccttaccac acatcaagac atcggagatg tacagcgtta ggatgcctaa catattgaac     540

ttctcatttg acttcttcta tgcaacgata ctcgtccttg caatctatgt cccaggtagc     600

ccacacatgt acaggtacat gcttggtcag cgaaagagag ctctctccaa atccaagagg     660

gaataa                                                                666


<210>  2
<211>  663
<212>  DNA
<213>  artificial

<220>
<223>  Recoded Arabidopsis PAS2 gene (PAS2YL)

<400>  2
atggccggct ttctgtctgt ggtgcgacga gtgtacctga ccctgtacaa ctggatcgtg      60

ttcgccggat gggcccaggt gctgtacctg gccatcacca ccctgaagga gaccggctac     120

gagaacgtgt acgacgccat cgagaagccc ctgcagctgg cccagaccgc cgctgtgctg     180

gagatcctgc acggcctggt gggactggtc cgatctcccg tgtctgccac cctgccccag    240

atcggctctc gactgttcct gacctggggc atcctgtact cgttccccga ggtgcgatct    300

cacttcctgg tgacctccct ggtgatctct tggtctatca ccgagatcat ccgatactcg    360

ttcttcggct tcaaggaggc cctgggcttc gccccctctt ggcacctgtg gctgcgatac    420

tcttctttcc tgctgctgta ccccaccggc atcacctctg aggtgggcct gatctacctg    480

gctctgcccc acatcaagac ctctgagatg tactctgtgc gaatgcccaa catcctgaac    540

ttctcgttcg acttcttcta cgccaccatc ctggtgctgg ctatctacgt gcccggctct    600

cctcacatgt accgatacat gctcggccag cgaaagcgag ccctgtctaa gtctaagcga    660

gag                                                                   663

<210>  3
<211>  662
<212>  DNA
<213>  Artificial

<220>
<223>  Arabidopsis PAS2 gene consensus sequence

<220>
<221>  misc_feature
<222>  (6)..(6)
<223>  n is a, c, g, or t

<220>
<221>  misc_feature
<222>  (15)..(15)
<223>  n is a, c, g, or t

<220>
<221>  misc_feature
<222>  (18)..(18)
<223>  n is a, c, g, or t

<220>
<221>  misc_feature
<222>  (21)..(21)
<223>  n is a, c, g, or t

<220>
<221>  misc_feature
<222>  (24)..(24)
<223>  n is a, c, g, or t

<220>
<221>  misc_feature
<222>  (27)..(27)
<223>  n is a, c, g, or t

<220>
<221>  misc_feature

<222>  (30)..(30)
<223>  n is a, c, g, or t

<220>
<221>  misc_feature
<222>  (39)..(39)
<223>  n is a, c, g, or t

<220>
<221>  misc_feature
<222>  (42)..(42)
<223>  n is a, c, g, or t

<220>
<221>  misc_feature
<222>  (45)..(45)
<223>  n is a, c, g, or t

<220>
<221>  misc_feature
<222>  (51)..(51)
<223>  n is a, c, g, or t

<220>
<221>  misc_feature
<222>  (60)..(60)
<223>  n is a, c, g, or t

<220>
<221>  misc_feature
<222>  (63)..(63)
<223>  n is a, c, g, or t

<220>
<221>  misc_feature
<222>  (66)..(66)
<223>  n is a, c, g, or t

<220>
<221>  misc_feature
<222>  (75)..(75)
<223>  n is a, c, g, or t

<220>
<221>  misc_feature
<222>  (81)..(82)
<223>  n is a, c, g, or t

<220>
<221>  misc_feature
<222>  (88)..(88)
<223>  n is a, c, g, or t

<220>
<221>  misc_feature
<222>  (90)..(90)
<223>  n is a, c, g, or t

<220>
<221>  misc_feature
<222>  (93)..(93)

<223>  n is a, c, g, or t

<220>
<221>  misc_feature
<222>  (96)..(96)
<223>  n is a, c, g, or t

<220>
<221>  misc_feature
<222>  (99)..(99)
<223>  n is a, c, g, or t

<220>
<221>  misc_feature
<222>  (102)..(103)
<223>  n is a, c, g, or t

<220>
<221>  misc_feature
<222>  (111)..(111)
<223>  n is a, c, g, or t

<220>
<221>  misc_feature
<222>  (114)..(114)
<223>  n is a, c, g, or t

<220>
<221>  misc_feature
<222>  (117)..(117)
<223>  n is a, c, g, or t

<220>
<221>  misc_feature
<222>  (120)..(120)
<223>  n is a, c, g, or t

<220>
<221>  misc_feature
<222>  (126)..(126)
<223>  n is a, c, g, or t

<220>
<221>  misc_feature
<222>  (129)..(129)
<223>  n is a, c, g, or t

<220>
<221>  misc_feature
<222>  (132)..(132)
<223>  n is a, c, g, or t

<220>
<221>  misc_feature
<222>  (141)..(141)
<223>  n is a, c, g, or t

<220>
<221>  misc_feature
<222>  (150)..(150)
<223>  n is a, c, g, or t

<220>
<221>  misc_feature
<222>  (153)..(153)
<223>  n is a, c, g, or t

<220>
<221>  misc_feature
<222>  (159)..(159)
<223>  n is a, c, g, or t

<220>
<221>  misc_feature
<222>  (162)..(162)
<223>  n is a, c, g, or t

<220>
<221>  misc_feature
<222>  (168)..(168)
<223>  n is a, c, g, or t

<220>
<221>  misc_feature
<222>  (174)..(174)
<223>  n is a, c, g, or t

<220>
<221>  misc_feature
<222>  (177)..(177)
<223>  n is a, c, g, or t

<220>
<221>  misc_feature
<222>  (180)..(180)
<223>  n is a, c, g, or t

<220>
<221>  misc_feature
<222>  (186)..(186)
<223>  n is a, c, g, or t

<220>
<221>  misc_feature
<222>  (189)..(189)
<223>  n is a, c, g, or t

<220>
<221>  misc_feature
<222>  (192)..(192)
<223>  n is a, c, g, or t

<220>
<221>  misc_feature
<222>  (195)..(196)
<223>  n is a, c, g, or t

<220>
<221>  misc_feature
<222>  (198)..(198)
<223>  n is a, c, g, or t

<220>
<221> misc_feature
<222> (201)..(201)
<223> n is a, c, g, or t

<220>
<221> misc_feature
<222> (204)..(205)
<223> n is a, c, g, or t

<220>
<221> misc_feature
<222> (211)..(211)
<223> n is a, c, g, or t

<220>
<221> misc_feature
<222> (219)..(219)
<223> n is a, c, g, or t

<220>
<221> misc_feature
<222> (222)..(222)
<223> n is a, c, g, or t

<220>
<221> misc_feature
<222> (228)..(228)
<223> n is a, c, g, or t

<220>
<221> misc_feature
<222> (231)..(231)
<223> n is a, c, g, or t

<220>
<221> misc_feature
<222> (237)..(237)
<223> n is a, c, g, or t

<220>
<221> misc_feature
<222> (243)..(243)
<223> n is a, c, g, or t

<220>
<221> misc_feature
<222> (246)..(246)
<223> n is a, c, g, or t

<220>
<221> misc_feature
<222> (249)..(250)
<223> n is a, c, g, or t

<220>
<221> misc_feature
<222> (252)..(252)
<223> n is a, c, g, or t

<220>

<221>  misc_feature
<222>  (255)..(255)
<223>  n is a, c, g, or t

<220>
<221>  misc_feature
<222>  (258)..(258)
<223>  n is a, c, g, or t

<220>
<221>  misc_feature
<222>  (261)..(261)
<223>  n is a, c, g, or t

<220>
<221>  misc_feature
<222>  (264)..(264)
<223>  n is a, c, g, or t

<220>
<221>  misc_feature
<222>  (276)..(276)
<223>  n is a, c, g, or t

<220>
<221>  misc_feature
<222>  (280)..(282)
<223>  n is a, c, g, or t

<220>
<221>  misc_feature
<222>  (285)..(285)
<223>  n is a, c, g, or t

<220>
<221>  misc_feature
<222>  (288)..(288)
<223>  n is a, c, g, or t

<220>
<221>  misc_feature
<222>  (294)..(294)
<223>  n is a, c, g, or t

<220>
<221>  misc_feature
<222>  (303)..(303)
<223>  n is a, c, g, or t

<220>
<221>  misc_feature
<222>  (306)..(306)
<223>  n is a, c, g, or t

<220>
<221>  misc_feature
<222>  (309)..(309)
<223>  n is a, c, g, or t

<220>
<221>  misc_feature

<222>  (312)..(312)
<223>  n is a, c, g, or t

<220>
<221>  misc_feature
<222>  (315)..(315)
<223>  n is a, c, g, or t

<220>
<221>  misc_feature
<222>  (318)..(318)
<223>  n is a, c, g, or t

<220>
<221>  misc_feature
<222>  (324)..(324)
<223>  n is a, c, g, or t

<220>
<221>  misc_feature
<222>  (327)..(330)
<223>  n is a, c, g, or t

<220>
<221>  misc_feature
<222>  (342)..(342)
<223>  n is a, c, g, or t

<220>
<221>  misc_feature
<222>  (348)..(348)
<223>  n is a, c, g, or t

<220>
<221>  misc_feature
<222>  (351)..(351)
<223>  n is a, c, g, or t

<220>
<221>  misc_feature
<222>  (354)..(354)
<223>  n is a, c, g, or t

<220>
<221>  misc_feature
<222>  (360)..(360)
<223>  n is a, c, g, or t

<220>
<221>  misc_feature
<222>  (372)..(372)
<223>  n is a, c, g, or t

<220>
<221>  misc_feature
<222>  (378)..(378)
<223>  n is a, c, g, or t

<220>
<221>  misc_feature
<222>  (381)..(381)

<223>  n is a, c, g, or t

<220>
<221>  misc_feature
<222>  (384)..(384)
<223>  n is a, c, g, or t

<220>
<221>  misc_feature
<222>  (390)..(390)
<223>  n is a, c, g, or t

<220>
<221>  misc_feature
<222>  (393)..(393)
<223>  n is a, c, g, or t

<220>
<221>  misc_feature
<222>  (396)..(396)
<223>  n is a, c, g, or t

<220>
<221>  misc_feature
<222>  (399)..(399)
<223>  n is a, c, g, or t

<220>
<221>  misc_feature
<222>  (406)..(406)
<223>  n is a, c, g, or t

<220>
<221>  misc_feature
<222>  (414)..(415)
<223>  n is a, c, g, or t

<220>
<221>  misc_feature
<222>  (421)..(426)
<223>  n is a, c, g, or t

<220>
<221>  misc_feature
<222>  (429)..(430)
<223>  n is a, c, g, or t

<220>
<221>  misc_feature
<222>  (432)..(433)
<223>  n is a, c, g, or t

<220>
<221>  misc_feature
<222>  (438)..(438)
<223>  n is a, c, g, or t

<220>
<221>  misc_feature
<222>  (444)..(444)
<223>  n is a, c, g, or t

<220>
<221>  misc_feature
<222>  (450)..(450)
<223>  n is a, c, g, or t

<220>
<221>  misc_feature
<222>  (457)..(459)
<223>  n is a, c, g, or t

<220>
<221>  misc_feature
<222>  (462)..(462)
<223>  n is a, c, g, or t

<220>
<221>  misc_feature
<222>  (465)..(465)
<223>  n is a, c, g, or t

<220>
<221>  misc_feature
<222>  (468)..(468)
<223>  n is a, c, g, or t

<220>
<221>  misc_feature
<222>  (471)..(471)
<223>  n is a, c, g, or t

<220>
<221>  misc_feature
<222>  (480)..(480)
<223>  n is a, c, g, or t

<220>
<221>  misc_feature
<222>  (483)..(484)
<223>  n is a, c, g, or t

<220>
<221>  misc_feature
<222>  (486)..(486)
<223>  n is a, c, g, or t

<220>
<221>  misc_feature
<222>  (489)..(489)
<223>  n is a, c, g, or t

<220>
<221>  misc_feature
<222>  (501)..(501)
<223>  n is a, c, g, or t

<220>
<221>  misc_feature
<222>  (504)..(504)
<223>  n is a, c, g, or t

<220>
<221> misc_feature
<222> (514)..(516)
<223> n is a, c, g, or t

<220>
<221> misc_feature
<222> (519)..(520)
<223> n is a, c, g, or t

<220>
<221> misc_feature
<222> (522)..(522)
<223> n is a, c, g, or t

<220>
<221> misc_feature
<222> (528)..(528)
<223> n is a, c, g, or t

<220>
<221> misc_feature
<222> (534)..(535)
<223> n is a, c, g, or t

<220>
<221> misc_feature
<222> (546)..(546)
<223> n is a, c, g, or t

<220>
<221> misc_feature
<222> (549)..(549)
<223> n is a, c, g, or t

<220>
<221> misc_feature
<222> (561)..(561)
<223> n is a, c, g, or t

<220>
<221> misc_feature
<222> (564)..(564)
<223> n is a, c, g, or t

<220>
<221> misc_feature
<222> (567)..(567)
<223> n is a, c, g, or t

<220>
<221> misc_feature
<222> (570)..(570)
<223> n is a, c, g, or t

<220>
<221> misc_feature
<222> (573)..(573)
<223> n is a, c, g, or t

<220>

<221>  misc_feature
<222>  (576)..(576)
<223>  n is a, c, g, or t

<220>
<221>  misc_feature
<222>  (579)..(579)
<223>  n is a, c, g, or t

<220>
<221>  misc_feature
<222>  (582)..(582)
<223>  n is a, c, g, or t

<220>
<221>  misc_feature
<222>  (588)..(588)
<223>  n is a, c, g, or t

<220>
<221>  misc_feature
<222>  (591)..(591)
<223>  n is a, c, g, or t

<220>
<221>  misc_feature
<222>  (594)..(594)
<223>  n is a, c, g, or t

<220>
<221>  misc_feature
<222>  (597)..(600)
<223>  n is a, c, g, or t

<220>
<221>  misc_feature
<222>  (603)..(603)
<223>  n is a, c, g, or t

<220>
<221>  misc_feature
<222>  (613)..(613)
<223>  n is a, c, g, or t

<220>
<221>  misc_feature
<222>  (615)..(615)
<223>  n is a, c, g, or t

<220>
<221>  misc_feature
<222>  (624)..(624)
<223>  n is a, c, g, or t

<220>
<221>  misc_feature
<222>  (627)..(627)
<223>  n is a, c, g, or t

<220>
<221>  misc_feature

<222>  (637)..(637)
<223>  n is a, c, g, or t

<220>
<221>  misc_feature
<222>  (642)..(642)
<223>  n is a, c, g, or t

<220>
<221>  misc_feature
<222>  (645)..(645)
<223>  n is a, c, g, or t

<220>
<221>  misc_feature
<222>  (648)..(648)
<223>  n is a, c, g, or t

<220>
<221>  misc_feature
<222>  (651)..(651)
<223>  n is a, c, g, or t

<220>
<221>  misc_feature
<222>  (654)..(654)
<223>  n is a, c, g, or t

<220>
<221>  misc_feature
<222>  (658)..(658)
<223>  n is a, c, g, or t

<220>
<221>  misc_feature
<222>  (660)..(660)
<223>  n is a, c, g, or t

<400>  3
atggcnggct ttctntcngt ngtncgncgn gtgtacctna cnctntacaa ntggatcgtn      60

ttngcnggat gggcncaggt nntgtacntn gcnatnacna cnntgaagga nacnggntan     120

gagaangtnt angacgccat ngagaagccn ctncagctng cncagacngc cgcngtnctn     180

gagatnctnc anggnntngt nggnntggtc ngatctccng tntctgcnac nctgccncag     240

atnggntcnn gnctnttnct nacntggggc atcctntacn nnttnccnga ggtncgatct     300

canttnctng tnacntcnct ggtnatnnnn tggtctatca cngagatnat ncgntactcn     360

ttcttcggct tnaaggangc nctnggcttn gcnccntcnt ggcacntgtg gctnngatac     420

nnnnnnttnn tnntgctnta cccnaccggn atcaccnnng angtnggnct natctacctn     480

gcnntnccnc acatcaagac ntcngagatg tacnnngtnn gnatgccnaa catnntgaac     540

ttctcnttng acttcttcta ngcnacnatn ctngtnctng cnatctangt nccnggnnnn     600

ccncacatgt acngntacat gctnggncag cgaaagngag cnctntcnaa ntcnaagngn     660

ga                                                                   662

<210>  4
<211>  619
<212>  DNA
<213>  artificial

<220>
<223>  Recoded Arabidopsis PAS2 gene

<400>  4
   ggtaccggat cccacaatgg tgctgtacct ggccatcacc accctgaagg agaccggcta      60
   cgagaacgtg tacgacgcca tcgagaagcc cctgcagctg gctcagaccg ccgctgtgct     120
   ggagatcctg cacggcctgg tgggactggt gcgatctccc gtgtctgcca ccctgcccca     180
   gatcggctct cgactgttcc tgacctgggg catcctgtac tcgttccccg aggtgcgatc     240
   tcacttcctg gtgacctccc tggtgatctc ttggtctatc accgagatca tccgatactc     300
   gttcttcggc ttcaaggagg ccctgggctt cgccccctct tggcacctgt ggctgcgata     360
   ctcttctttc ctgctgctgt accccaccgg catcacctct gaggtgggcc tgatctacct     420
   cgctctgcct cacatcaaga cctctgagat gtactctgtg cgaatgccca acatcctgaa     480
   cttctcgttc gacttcttct acgccaccat cctggtgctg gctatctacg tgcccggctc     540
   tcctcacatg taccgataca tgctgggcca gcgaaagcga gccctgtcta agtctaagcg     600
   agagtagcct agggagctc                                                  619

<210>  5
<211>  196
<212>  PRT
<213>  artificial

<220>
<223>  Recoded Arabidopsis PAS2 protein

<400>  5

Met Val Leu Tyr Leu Ala Ile Thr Thr Leu Lys Glu Thr Gly Tyr Glu
1               5                   10                  15

Asn Val Tyr Asp Ala Ile Glu Lys Pro Leu Gln Leu Ala Gln Thr Ala
            20                  25                  30

Ala Val Leu Glu Ile Leu His Gly Leu Val Gly Leu Val Arg Ser Pro
        35                  40                  45

Val Ser Ala Thr Leu Pro Gln Ile Gly Ser Arg Leu Phe Leu Thr Trp
    50                  55                  60

Gly Ile Leu Tyr Ser Phe Pro Glu Val Arg Ser His Phe Leu Val Thr
65                  70                  75                  80

Ser Leu Val Ile Ser Trp Ser Ile Thr Glu Ile Ile Arg Tyr Ser Phe
                85                  90                  95

Phe Gly Phe Lys Glu Ala Leu Gly Phe Ala Pro Ser Trp His Leu Trp
            100                 105                 110

Leu Arg Tyr Ser Ser Phe Leu Leu Leu Tyr Pro Thr Gly Ile Thr Ser
        115                 120                 125

Glu Val Gly Leu Ile Tyr Leu Ala Leu Pro His Ile Lys Thr Ser Glu
    130                 135                 140

Met Tyr Ser Val Arg Met Pro Asn Ile Leu Asn Phe Ser Phe Asp Phe
145                 150                 155                 160

Phe Tyr Ala Thr Ile Leu Val Leu Ala Ile Tyr Val Pro Gly Ser Pro
                165                 170                 175

His Met Tyr Arg Tyr Met Leu Gly Gln Arg Lys Arg Ala Leu Ser Lys
            180                 185                 190

Ser Lys Arg Glu
        195

<210>  6
<211>  25
<212>  DNA
<213>  artificial

<220>
<223>  LRO1-ver1 primer

<400>  6
ccacggagac tggaacagat gtcgg                                          25

<210>  7
<211>  23
<212>  DNA
<213>  artificial

<220>
<223>  LRO1-P1 primer

<400>  7
ggatcccagt gctctagact gtc                                            23

<210>  8

<211>  46
<212>  DNA
<213>  artificial

<220>
<223>  LRO1-P2 primer

<400>  8
gctagggata acagggtaat gcgcggtagc tgagacatgt cgcgtg                    46


<210>  9
<211>  44
<212>  DNA
<213>  artificial

<220>
<223>  LRO1-T1 primer

<400>  9
gcattaccct gttatcccta gcgcgttcgt cctctcatga ttcc                      44


<210>  10
<211>  24
<212>  DNA
<213>  artificial

<220>
<223>  LRO1-T2 primer

<400>  10
ccaaacatag tcatttgcgg atcc                                            24


<210>  11
<211>  22
<212>  DNA
<213>  artificial

<220>
<223>  LRO1-ver2

<400>  11
ccaagggacc gtctggccaa ac                                              22


<210>  12
<211>  25
<212>  DNA
<213>  artificial

<220>
<223>  DGA1-ver1

<400>  12
cggacacctc ttttatgctg cgggc                                           25


<210>  13
<211>  23

<212>  DNA
<213>  artificial

<220>
<223>  DGA1-P1 primer

<400>  13
ggcggatcct ggtgcatttt tgc                                              23


<210>  14
<211>  45
<212>  DNA
<213>  artificial

<220>
<223>  DGA1-T1 primer

<400>  14
gctagggata acagggtaat gcgcaaactc atctggggga gatcc                      45


<210>  15
<211>  45
<212>  DNA
<213>  artificial

<220>
<223>  DGA1-P2 primer

<400>  15
gcattaccct gttatcccta gcgagcttat cagtcacggt ccacg                      45


<210>  16
<211>  21
<212>  DNA
<213>  artificial

<220>
<223>  DGA1-T2 primer

<400>  16
ccatagaggt gtccccaaac g                                                21


<210>  17
<211>  23
<212>  DNA
<213>  artificial

<220>
<223>  DGA1-ver2 primer

<400>  17
cccccaagca taccgaccgt cgc                                              23


<210>  18
<211>  23
<212>  DNA

<213>  artificial

<220>
<223>  61start primer

<400>  18
cttatatacc aaagggatgg gtc                                              23


<210>  19
<211>  23
<212>  DNA
<213>  artificial

<220>
<223>  61stop primer

<400>  19
gtagatagtt gaggtagaag ttg                                              23


<210>  20
<211>  21
<212>  DNA
<213>  artificial

<220>
<223>  TEFstart primer

<400>  20
gggtataaaa gaccaccgtc c                                                21